# EUROPEAN PATENT APPLICATION

(11) **EP 2 221 313 A1**
(43) Date of publication of application: **25.08.2010**
(21) Application number: 10152150.8
(22) Date of filing: 29.01.2010
(51) Int. Cl.: C07J 9/00, C07J 75/00

(54) **Process for the preparation of ursodeoxycholic acid disodium 3,7-disulfate**

(30) Priority: 19.02.2009 IT MI20090220
(71) Applicant: PRODOTTI CHIMICI E ALIMENTARI SPA, 15060 Basaluzzo (Alessandria) (IT)
(72) Inventor: Galdi, Gianluca, I-16030 Pieve Ligure (Genova) (IT); Parenti, Massimo, I-15067 Novi Ligure (Alessandria) (IT); Zuccotti, Pierfranca, I-15068 Pozzolo Formigaro (Alessandria) (IT)
(74) Representative: Pistolesi, Roberto

(57) **Abstract**

Process for preparing the disodium salt of ursodeoxycholic acid 3,7-disulfate comprising the following steps: a) reaction of ursodeoxycholic acid with a complex of trimethylamine-sulphuric anhydride, to obtain the ursodeoxycholic acid 3,7-disulfate di-trimethylammonium reaction intermediate; b) treatment of the di-trimethylammonium salt of ursodeoxycholic acid 3,7-disulfate with organic or inorganic sodium bases to obtain the tri-sodium salt of ursodeoxycholic acid 3,7-disulfate; c) treatment with an inorganic acid in presence of isopropanol up to a pH comprised in the range between 3.0 and 4.5, to obtain the disodium salt of ursodeoxycholic acid 3,7-disulfate in solution; d) removal of the salts present and obtaining the disodium salt ofursodeoxycholic acid 3,7-disulfate.

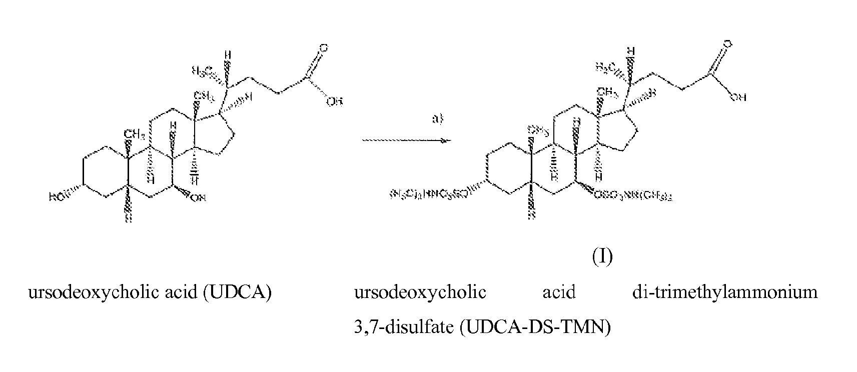

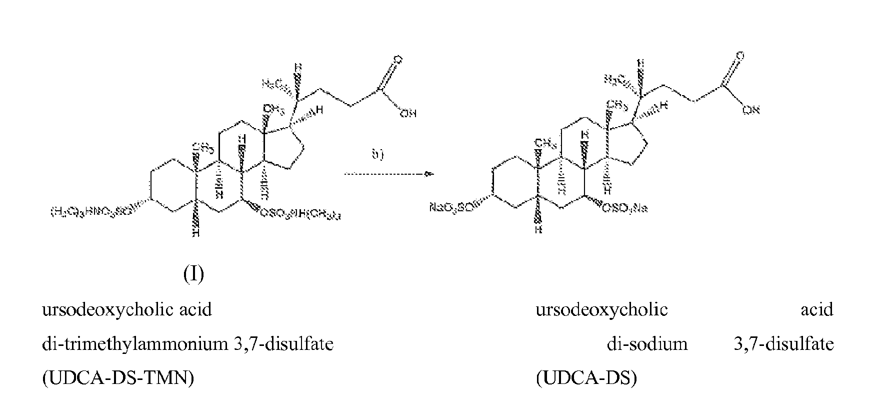

## Description

The present invention relates to a new process for the synthesis of the disodium salt of ursodeoxycholic acid 3,7-disulfate and to a new intermediate of said synthesis.

### BACKGROUND ART

Ursodeoxycholic acid (hereinafter referred as UDCA), a well known secondary biliary acid, being present in the intestine as a metabolic by-product of intestinal bacteria, is used in therapy due to its hepatoprotective activities, since it is capable of increasing in human beings the solubilising capacity of bile with respect to cholesterol, transforming lithogenic bile into non-lithogenic bile. Treatment with ursodeoxycholic acid therefore determines the formation of bile unsaturated with cholesterol and richer in biliary salts suitable for solubilisation thereof, also facilitating a regular flow of bile and the emptying of gallbladder.

A limitation on the use of UDCA lies in its poor solubility in water, which limits the formulation thereof to oral solid forms, such as tablets and capsules.

It is known in the art that UDCA derivatives, such as sulphates, allow to overcome the abovementioned drawback.

Documents EP117570, WO97/18816, WO03/066657 and W02004/092193, herein incorporated by reference, thus describe ursodeoxycholic acid 3,7-disulfate (hereinafter referred as UDCA-DS) and salts thereof, in particular di- and tri-sodium salts, which are highly soluble in water, thus allowing its formulation in liquid preparations, while maintaining the pharmacological properties unaltered.

Furthermore, these documents describe some synthesis routes of UDCA-DS and salts thereof, which, however, have various disadvantages.

EP117570 and WO97/18816 use for the sulfation of UDCA toxic or hazardous reagents such sulphuric anhydride-pyridine and chlorosulfonic acid-pyridine complexes, which require complex process steps and specific technical measures to provide safe conditions.

Furthermore, the prior art syntheses do not allow to obtain a product free of undesired by-products, such as UDCA-DS methyl ester which is formed using the methods described in WO03/066657 and W02004/092193.

Therefore, there arises the need of a synthesis route of the sodium salt of UDCA-DS in high yields, capable of providing such compound with a high degree of purity, without using toxic or hazardous reagents.

### SUMMARY OF THE INVENTION

Thus, the object of the present invention is a synthesis route of the disodium salt of UDCA-DS allowing to overcome the drawbacks of the prior art.

As a matter of fact, the applicant has surprisingly found that the use of reagents such as the complex of trimethylamine and sulphuric anhydride allows to obtain a stable compound of UDCA, i.e. salt of di-trimethylammonium of ursodeoxycholic acid 3,7-disulfate (hereinafter referred as UDCA-DS-TMN), in high yields. Such compound is useful as an intermediate in the synthesis of di-sodium salt of UDCA-DS. As a matter of fact, the subsequent treatment of the UDCA-DS-TMN salt with organic or inorganic sodium bases, allows to obtain the tri-sodium salt of UDCA-DS. Hence, partial acidification with an inorganic acid in the presence of an alcohol, preferably isopropanol, allows to obtain the disodium salt of UDCA-DS in high yields and with a high degree of purity.

Thus, the object of the present invention is also the intermediate obtained from the reaction of UDCA with the complex of trimethylamine-sulphuric anhydride, i.e. the UDCA-DS-TMN salt.

### DESCRIPTION OF THE FIGURES

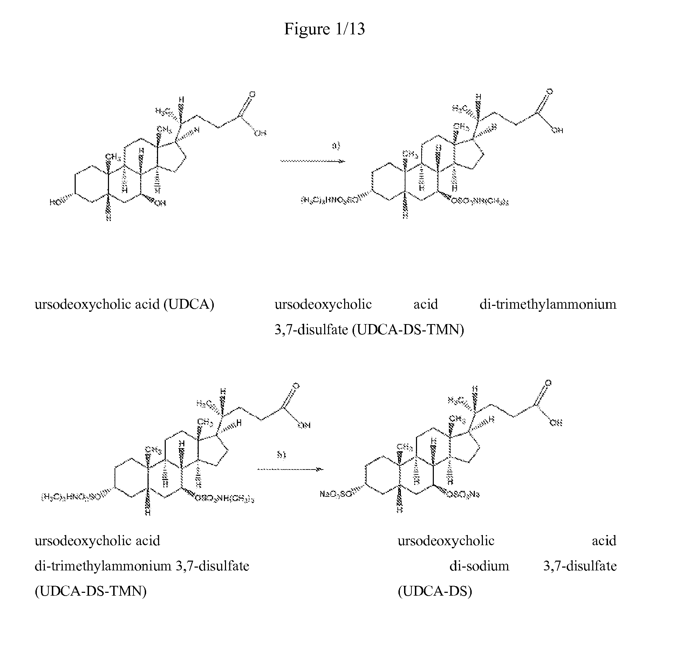

Figure 1: the synthesis route of the disodium salt of ursodeoxycholic acid 3,7-disulfate (disodium salt of UDCA-DS) is shown.

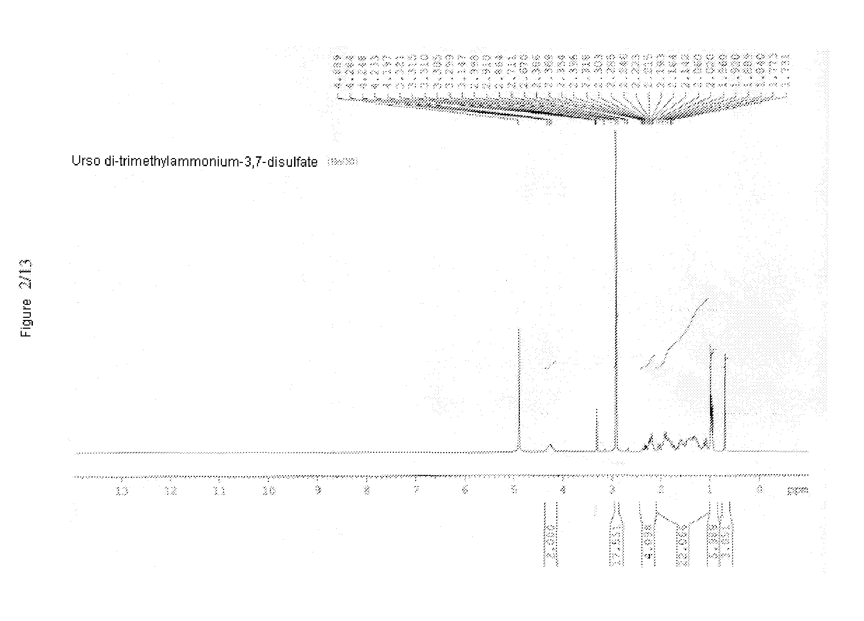

Figure 2 - ¹HNMR characterisation of the UDCA-DS-TMN compound

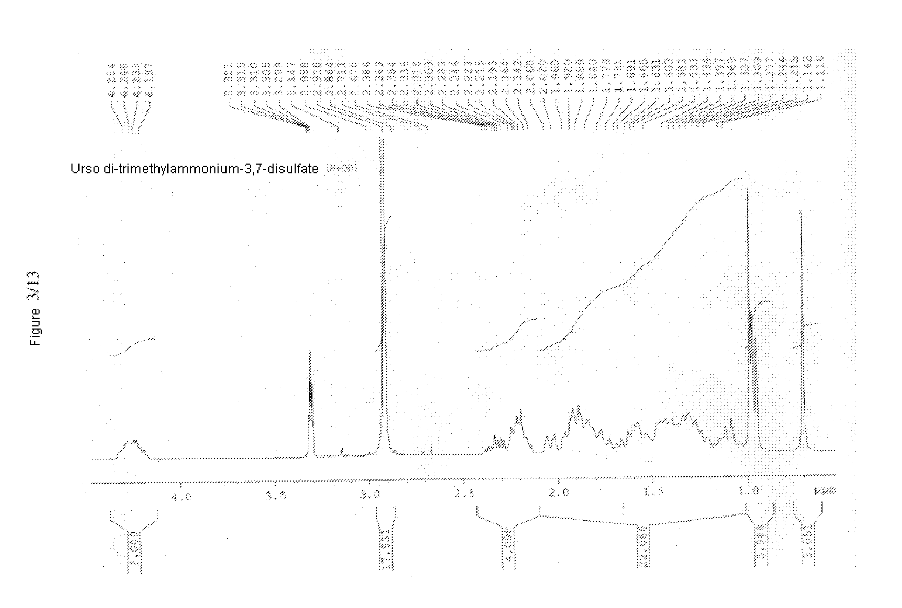

Figure 3 - ¹HNMR characterisation of the UDCA-DS-TMN compound

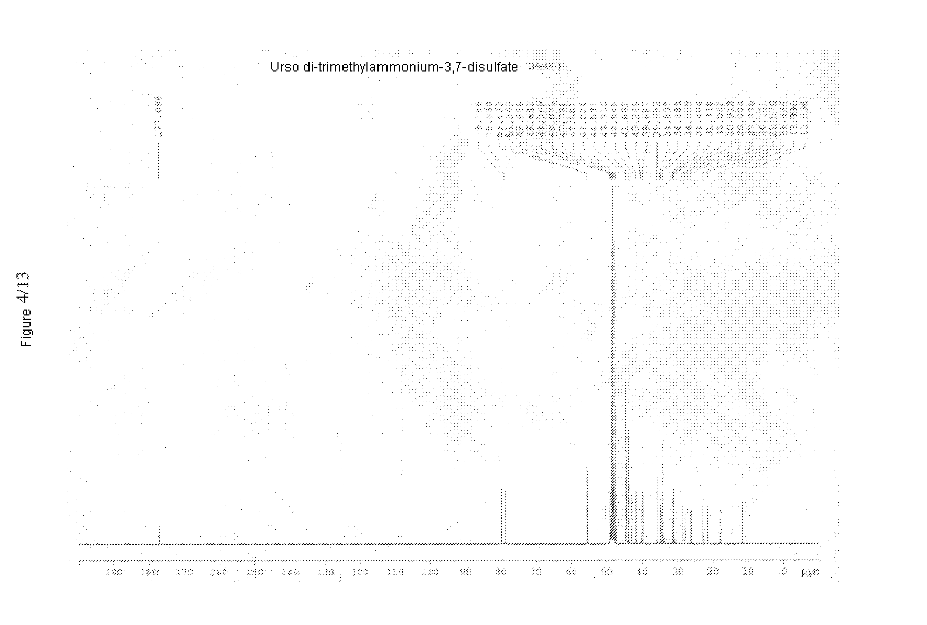

Figure 4 - ¹³CNMR characterisation of the UDCA-DS-TMN compound

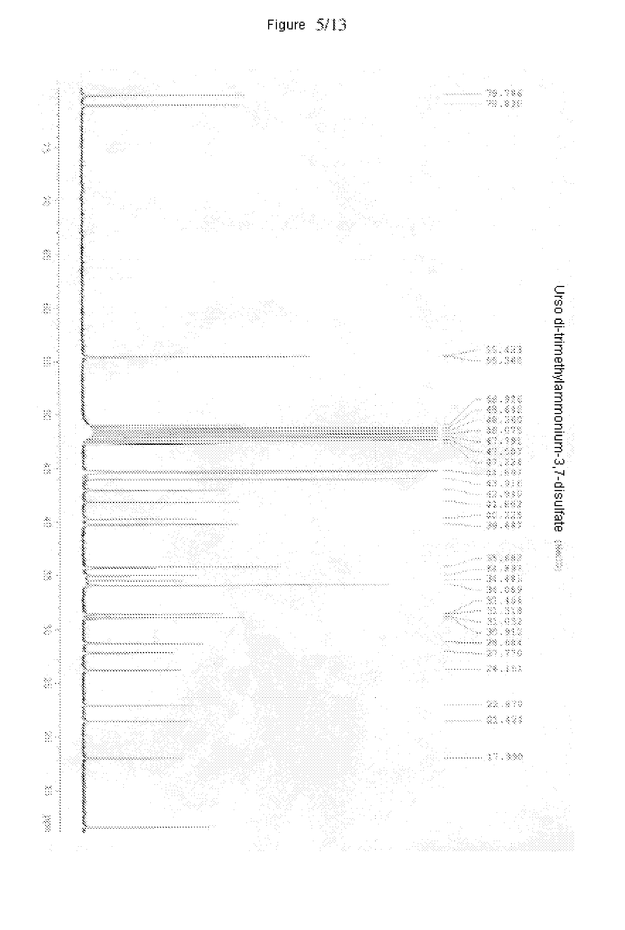

Figure 5 - ¹³CNMR characterisation of the UDCA-DS-TMN compound

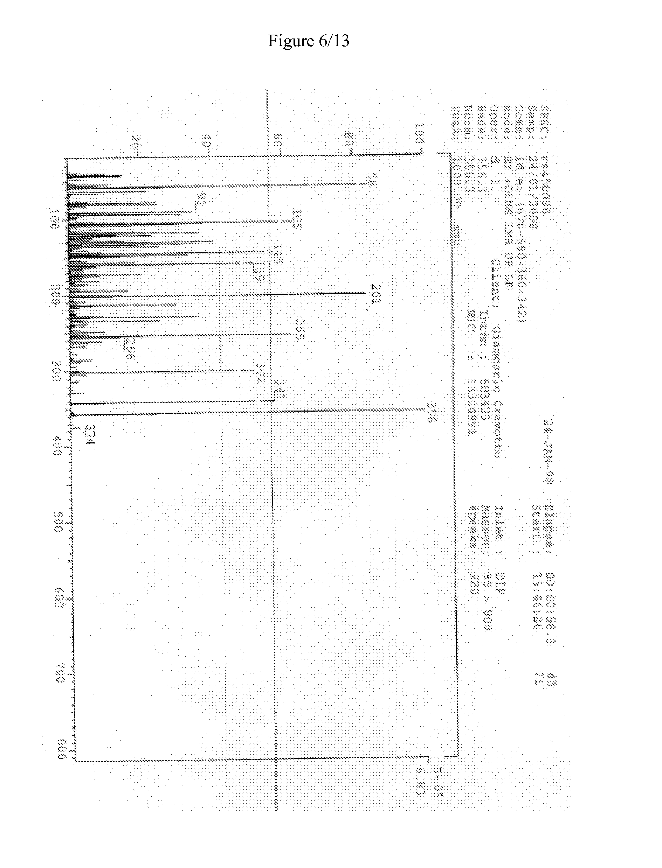

Figure 6: MS characterisation of the UDCA-DS-TMN compound

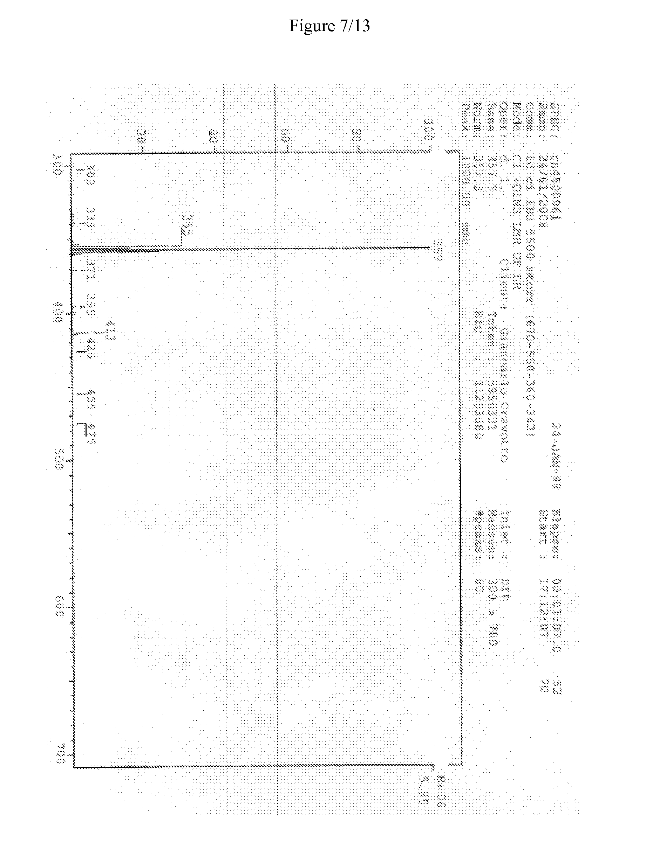

Figure 7: MS characterisation of the UDCA-DS-TMN compound

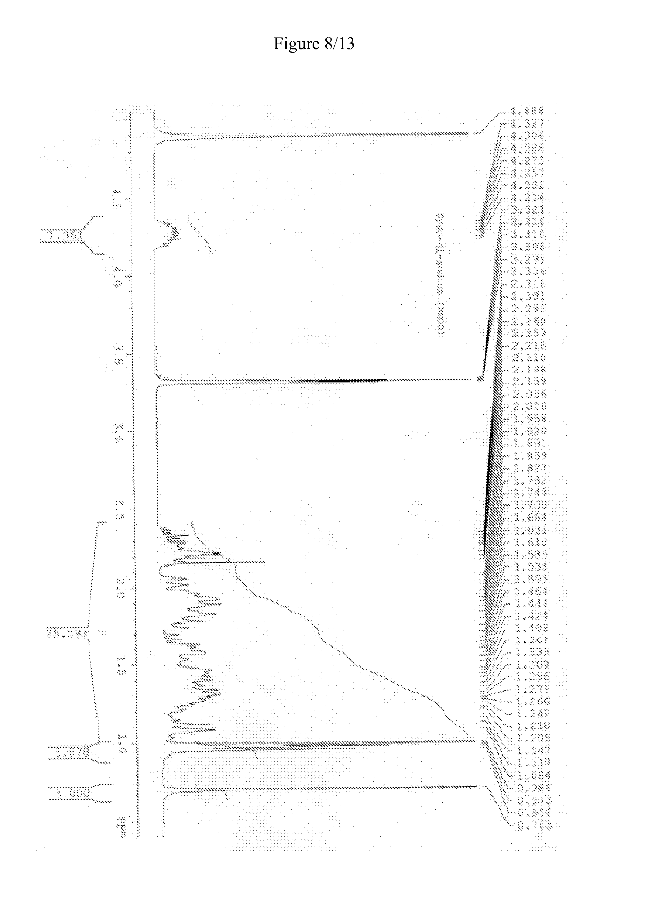

Figure 8: ¹HNMR characterisation of the UDCA-DS di-sodium salt compound

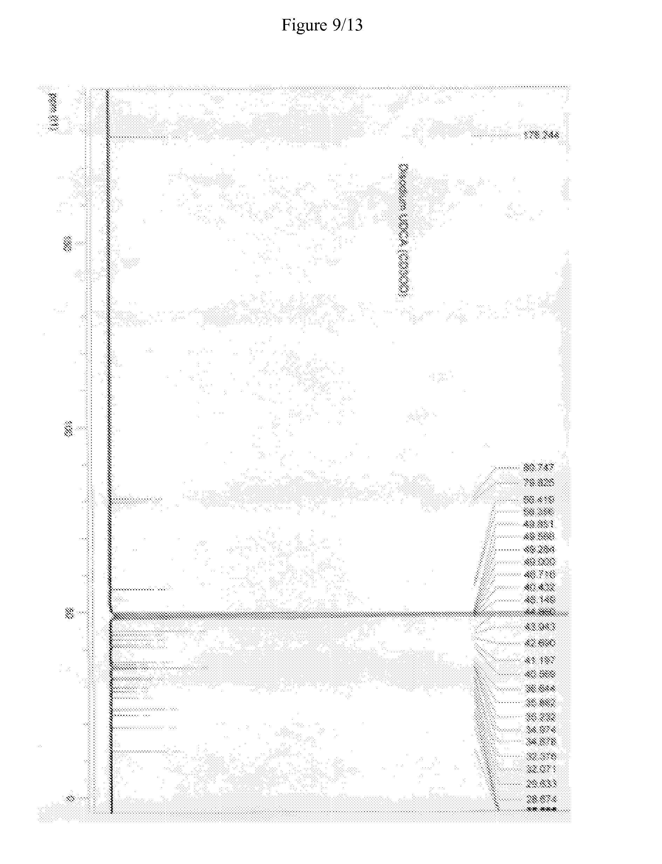

Figure 9: ¹³C-NMR characterisation of the UDCA-DS di-sodium salt compound

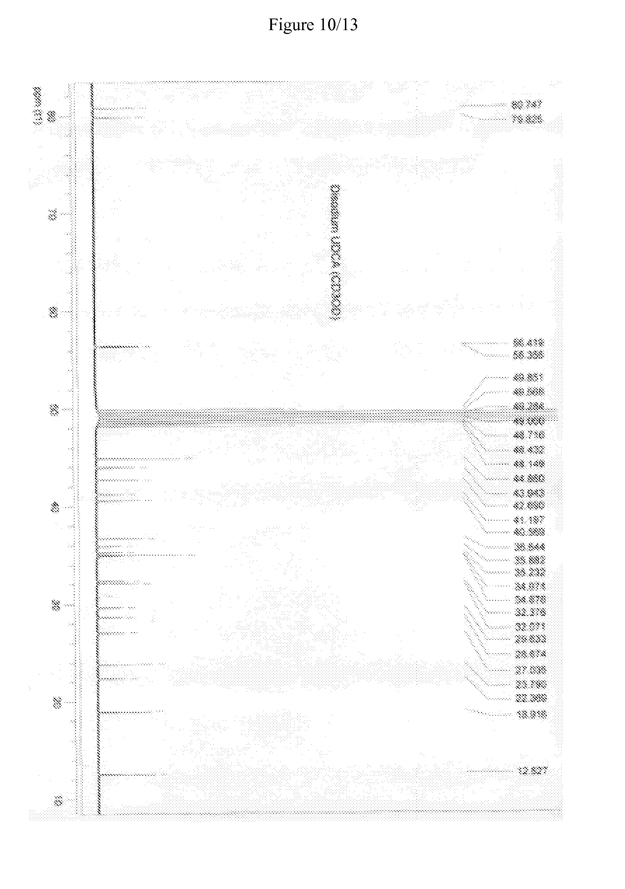

Figure 10: ¹³C-NMR characterisation of the UDCA-DS di-sodium salt compound

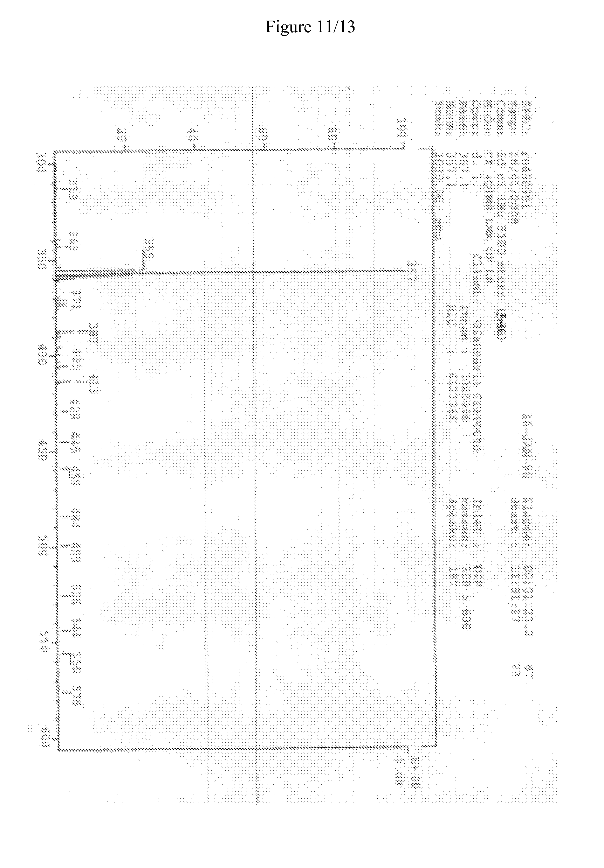

Figure 11: CI-MS characterisation of the UDCA-DS di-sodium salt compound

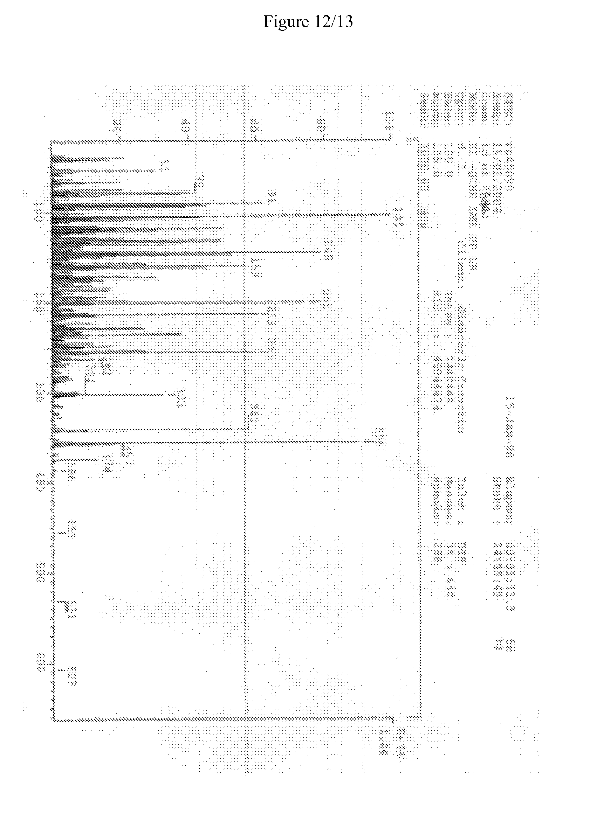

Figure 12: EI-MS characterisation of the UDCA-DS di-sodium salt compound

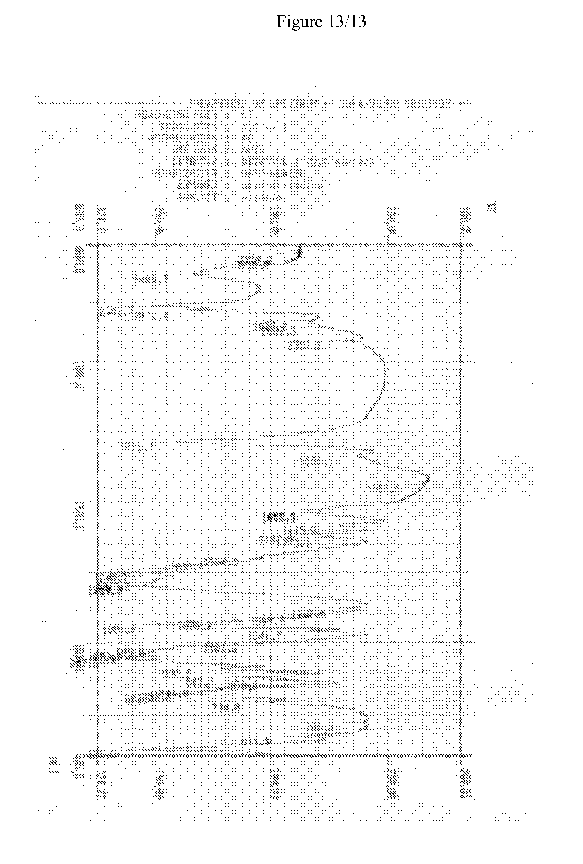

Figure 13: IR characterisation of the UDCA-DS di-sodium salt compound

### DESCRIPTION OF THE INVENTION

Thus, an aspect of the present invention is a process for the synthesis of the disodium salt of UDCA-DS, comprising the following steps:
a) reaction of ursodeoxycholic acid with a complex of trimethylamine-sulphuric anhydride, to obtain the di-trimethylammonium salt of ursodeoxycholic acid 3,7-disulfate;
b) treatment of the di-trimethylammonium salt of ursodeoxycholic acid 3,7-disulfate with organic or inorganic sodium bases to obtain the tri-sodium salt of UDCA-DS;
c) treatment of the tri-sodium salt of UDCA-DS with an inorganic acid in presence of isopropanol up to a pH comprised in the range between 3.0 and 4.5, to obtain the disodium salt of ursodeoxycholic acid 3,7-disulfate in solution.
   The process object of the present invention, may optionally comprise the following step:
d) removal of the sodium salt of the inorganic acid used at step c) and obtaining the disodium salt of ursodeoxycholic acid 3,7-disulfate.

The reaction step a) is preferably carried out using a complex between trimethylamine and sulphuric anhydride, which, surprisingly, allows to obtain the intermediate UDCA-DS-TMN as a crystalline salt. As a matter of fact, the use of the complex of sulphuric anhydride with other amines, such as pyridine or triethylamine and also the complex with DMF, did not allow to obtain a crystalline intermediate, which can be isolated.

The preferred molar ratios of UDCA to complex between trimethylamine and sulphuric anhydride are comprised between 1:2.1 and 1:2.3. In particular the preferred ratio is 1:2.2.

Step a) is preferably carried out using an aprotic polar organic solvent, even more preferably dimethylformamide (DMF) or dimethylacetamide (DMA), at a temperature comprised between 40 and 90° C. The product obtained in solution, i.e. the UDCA-DS-TMN reaction intermediate, is preferably precipitated, by means of crystallisation in a C₃-C₆ ketone, even more preferably, acetone. Salification with the organic and inorganic sodium bases of step b) is carried out with sodium methylate, with sodium ethylate or sodium hydroxide, preferably with sodium hydroxide in a C₁-C₄ alcohol, even more preferably in methanol at a temperature comprised between 20 and 30° C.

In step c), partial acidification is carried out up to a pH comprised between 3.0 and 4.5, under the action of inorganic acids such as phosphoric acid, hydrochloric acid, sulphuric acid, acetic acid, hydrochloric acid being preferably used.

The preferred solvent used in the partial acidification step is isopropyl alcohol. As a matter of fact, it was surprisingly found that obtaining the disodium salt of UDCA-DS through partial acidification of the tri-sodium salt of UDCA-DS in isopropyl alcohol, allows to obtain such disodium salt with a high degree of purity, since there is no formation of the by-product of carboxyl esterification in position 24, between said alcohols and UDCA-DS. On the contrary, in presence of other alcohols, such as for example, methyl alcohol, there is the formation of the esterification by-product of UDCA-DS. Thus, with methanol, one obtains a less pure product, which has therefore a lower yield and quality.

Lastly, in step d), the present salts are removed by filtration, i.e. the sodium salts of the inorganic acids used in step c) are removed, then concentration under vacuum is carried out until dry, then acetone is added for the precipitation of the final product, possibly with addition of a small amount of water, about 25-30% with respect to the weight of the tri-sodium salt of UDCA-DS.

A further aspect of the present invention is represented by the UDCA-DS-TMN compound, of formula (I): 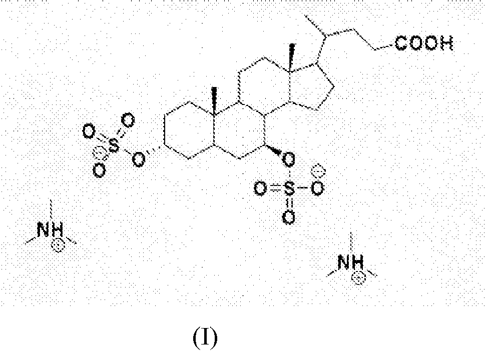 obtained as an intermediate in the synthesis process of the disodium salt of UDCA-DS.

An object of the present invention is also the use of the UDCA-DS-TMN compound as an intermediate.

The following examples illustrate the invention, without limiting it in any way.

### EXAMPLES

### Example 1

### Synthesis of UDCA-DS-TMN.

20 g of pure UDCA and 30 g of dimethylformamide (DMF) were charged into a glass flask. The solution is heated to 40-50° C, then 15.5 g of the sulphuric anhydride-trimethylamine complex are added. The ratio between UDCA and sulphuric anhydride-trimethylamine complex is 1:2.2. A suspension is obtained, which is heated to 80-90° C. After about 2-5 minutes the suspension turns into a solution. The solution is thus maintained at this temperature for 1 hour, then it is cooled using water.

When the solution reaches room temperature, 200 ml of acetone are added, over about 15 minutes, then it is left under stirring for 4 hours.

The suspension that is obtained is filtered and the product cake is washed with 50 ml of acetone, obtaining 40.6 g of wet product. The product is dried at 50° C, at room pressure, obtaining 31.3 g of dry product, stoichiometric yield 91.6%, with melting point comprised between 186.5° C and 189.2° C.

The product was characterised with thin layer chromatography (TLC), NMR and mass spectrometry.

Eluent used in the TLC: mixture of the solvents ethyl acetate/acetone/methanol/acetic acid/water at the following ratios: 50:35:10:10:0.5.
**¹H NMR** (CD₃OD): 4.24 (br, 2H, H-3α, H-7β), 2.91 (s, 18H), 2.38-2.14 (m, 4H), 2.06-1.11 (m, 22H) 0.98 (m, 6H, Me-21, Me-19), 0.70 (s, 3H, Me-18). Solvent residual peak: 4.99 (H₂O), 3.31 (CD₃OD).
**¹²C NMR** (CD₃OD): 177.0 (C24), 79.7 (C7), 78.8 (C3), 55.4, 44.5 (N-CH₃), 43.9, 42.9, 41.8, 40.2, 39.6, 35.6, 34.8, 34.4, 34.0, 31.4, 31.0, 28.6, 27.7, 26.1, 22.8 (C19), 21.4 (C11), 17.9 (C21), 11.5 (C18).
**EI-MS:** 374, 356 [M⁺-2NaHSO₄] (100), 341, 302, 255, 201, 159, 145, 105, 91, 58.
**CI-MS** (isobutane) m/z (%): 413, 395, 371, 357 [MH-2NaHSO₄]⁺ (100), 355, 339.

### Example 2

### Synthesis of the tri-sodium salt of UDCA-DS.

200 g of methanol and 11.9 g of sodium hydroxide were charged into a 1 litre glass flask. Stirring at room temperature is maintained until complete solution is obtained. Then, 50 g of UDCA-DS-TMN were charged into a 0.5 litre flask and the previously prepared sodium hydroxide solution in methanol is added thereto.

The solution obtained is placed under vacuum and stirred, keeping the internal temperature below 30° C, for about 1 hour.

Then, still under vacuum, the solution is dried.
150 g of acetone and 150 g of isopropanol are added onto the dry residue. The suspension is kept under stirring at room temperature for about 3 hours, then it is filtered, washing the cake on the filter with 50 ml of acetone.

The product is then dried at room pressure, thus obtaining 50 g of dry product, equivalent to the stoichiometric quantity.

### Example 3

### Synthesis of the disodium salt of UDCA-DS.

30 g of tri-sodium salt of UDCA-DS and 180 ml of isopropyl alcohol are charged into a 0.5 litre glass flask.

The suspension is refluxed, at about 81°C. The product does not dissolve. 51.5 ml of water are then added at reflux and the product dissolve in solution. The pH of the solution is 10.5.

The solution is then cooled up to room temperature and the pH is adjusted to about 3.0, constant with 4 ml of hydrochloric acid, 37%.

The suspension is stirred for about 15-20 minutes and then filtered, removing the salts that precipitate (1.4 g).

The filtered clear solution is concentrated under vacuum until dry. 660 ml of acetone are added to the dry residue.

The suspension is stirred for about 3 hours, then the cake is filtered and washed with 50 ml of acetone.

The product is dried at 50° C, at room pressure and a dry yield of 24 g is obtained, equivalent to 83% of the stoichiometric value.

A TLC is carried out using a fresh eluent to verify the absence of the isopropyl ester of UDCA-DS, which accordingly is not observed.
Eluent: chloroform/methanol/acetic acid/water at 65:24:15:9 ratio
**¹H NMR** (CD₃OD): 4.27 (br, 2H, H-3α, H-7β), 2.40-1.08 (M, 26H), 0.98 (d, 3H,Me-21), 0.95 (s, 3H, Me-19) 0.70 (s, 3H, Me-18). Solvent residual peak: 4.88 (H₂O), 3.31 (CD₃OD).
**¹²C NMR** (CD₃OD): 178.2 (C24), 80.7 (C7), 79.8 (C3), 56.4 (C17), 56.3 (C14), 44.8 (C13), 43.9 (C5), 42.6 (C8), 41.1 (C12), 40.5 (C9), 36.6 (C6), 35.8 (C4), 35.2 (C20), 34.9 (C1), 34.8 (C10), 32.3 (C22), 32.0 (C23), 29.6 (C2), 28.16 (C16), 27.0 (C15), 23.7 (C19), 22.3 (C11), 18.9 (C21), 12.5 (C 18).

**EI-MS:** 455, 386, 374, 357, 356 [M⁺-2NaHSO₄], 341, 302, 255, 213, 201, 159, 145, 105 (100), 91, 79, 55.
**CI-MS** (isobutane) m/z (%): 413, 405, 387, 371, 357 [MH-2NaHSO₄]⁺ (100), 355, 343, 313.
**IR** (KBr): ν = 3489 (OH), 2943, 2675, 1711 (-COOH), 1655, 1466, 1387, 1263, 1217, 1064, 983, 823 cm⁻¹.

## Claims

1. Process for preparing the di-sodium salt of ursodeoxycholic acid 3,7-disulfate comprising the following steps:
a) reaction of ursodeoxycholic acid with a complex of trimethylamine and sulphuric anhydride, to obtain the di-trimethylammonium salt of ursodeoxycholic acid 3,7-disulfate;
b) treatment of the di-trimethylammonium salt of ursodeoxycholic acid 3,7-disulfate with organic or inorganic sodium bases to obtain the tri-sodium salt of ursodeoxycholic acid 3,7-disulfate;
c) treatment of the tri-sodium salt of ursodeoxycholic acid 3,7-disulfate with an inorganic acid in presence of isopropanol up to a pH comprised in the range between 3.0 and 4.5, obtaining the disodium salt of ursodeoxycholic acid 3,7-disulfate in solution.

2. Process according to claim 1, wherein the molar ratio between ursodeoxycholic acid and the complex between trimethylamine and sulphuric anhydride in step a) is comprised in the range between 1:2.1 and 1:2.3.

3. Process according to claim 2, wherein the molar ratio between ursodeoxycholic acid and the complex between trimethylamine and sulphuric anhydride in step a) is 1:2.2.

4. Process according to claim 1, wherein step a) is carried out using an aprotic polar solvent.

5. Process according to claim 4, wherein the aprotic solvent used is dimethylformamide or dimethylacetamide.

6. Process according to claim 1, wherein the di-trimethylammonium salt of ursodeoxycholic acid 3,7-disulfate is precipitated in step a) by means of crystallisation in a C₃-C₆ ketone.

7. Process according to claim 6, wherein the ketone used is acetone.

8. Process according to claim 1, wherein the organic sodium base of step b) is sodium methylate or sodium ethylate.

9. Process according to claim 1, wherein the inorganic sodium base of step b) is sodium hydroxide.

10. Process according to claim 1, wherein said organic or inorganic sodium bases are used in presence of a C₁-C₄ alcoholic solvent.

11. Process according to claim 10, wherein said alcoholic solvent is methanol.

12. Process according to claim 1, wherein the temperature of step b) is comprised between 20 and 30° C.

13. Process according to claim 1, wherein the inorganic acid used in step c) is phosphoric acid, hydrochloric acid, sulphuric acid or acetic acid.

14. Process according to claim 13, wherein the inorganic acid used is hydrochloric acid.

15. Process according to claim 1, further comprising the following step:
d) removal of the sodium salt of the inorganic acid used at step c) and obtaining the disodium salt of ursodeoxycholic acid 3,7-disulfate in crystalline form.

16. Process according to claim 15, wherein the removal of salts occurs by filtration.

17. Di-trimethylammonium of ursodeoxycholic acid 3,7-disulfate of formula:

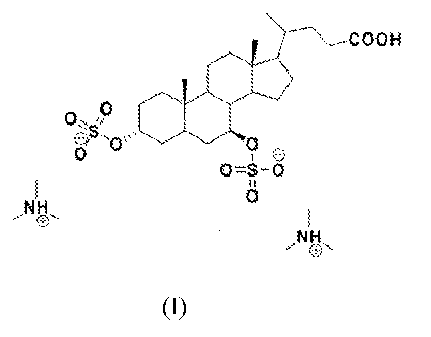

18. Use of the compound of formula (I) according to claim 17, as a reaction intermediate.
